## Europäisches Patentamt

## European Patent Office

## Office européen des brevets

(19)

(11) Veröffentlichungsnummer: **0 1 1 3 684**
**B1**

(12) **EUROPÄISCHE PATENTSCHRIFT**

(45) Veröffentlichungstag der Patentschrift:
**13.05.87**

(51) Int. Cl.⁴: **A 61 F 11/00**, G 10 K 11/28

(21) Anmeldenummer: **84101180.2**

(22) Anmeldetag: **06.02.84**

(54) **Hörgerät mit zwei über die Ohren zu stülpenden etwa muschelförmigen Schallauffangtrichtern.**

(30) Priorität: **15.07.83 DE 3325532**

(43) Veröffentlichungstag der Anmeldung:
**18.07.84 Patentblatt 84/29**

(45) Bekanntmachung des Hinweises auf die Patenterteilung:
**13.05.87 Patentblatt 87/20**

(84) Benannte Vertragsstaaten:
**AT BE CH FR GB IT LI LU NL SE**

(56) Entgegenhaltungen:
**DE - C - 280 653**
**FR - A - 1 515 905**
**GB - A - 623 791**
**US - A - 1 708 257**
**US - A - 1 820 107**
**US - A - 2 469 254**

(73) Patentinhaber: **Mertens, Hans Adolf, Am Schinnergraben 81, D-6500 Mainz 42 (DE)**

(72) Erfinder: **Mertens, Hans Adolf, Am Schinnergraben 81, D-6500 Mainz 42 (DE)**

(74) Vertreter: **Kodron, Rudolf S., Dipl.-Ing., Adam-Karrillon-Strasse 30, D-6500 Mainz (DE)**

## Beschreibung

Die Erfindung betrifft ein Hörgerät mit zwei über die Ohren zu stülpenden, einstückig hergestellten Schallauffangtrichtern aus Kunststoff gemäss GB-A-623 791.

Durch die US-Patentschrift 3 938 616 beispielsweise ist ein vergleichbares Hörgerät bekanntgeworden, das jedoch zwei Hauptnachteile besitzt, die einer Anwendung dieses Gerätes entgegenstehen.

Der erste Nachteil besteht in der komplizierten Bauweise und in der hohen Anzahl von fünf Bauteilen, die ineinandergeführt und relativ zueinander verschiebbar sind. Die fünf Bauteile umfassen einen über den Kopf reichenden Tragbügel, zwei in den Tragbügelenden verschiebbare Verlängerungsstäbe und die zwei in den Verlängerungsstäben ebenfalls verschiebbar geführten Schallauffangtrichter.

Der zweite wesentliche Nachteil besteht darin, dass die gesamte fünfteilige Konstruktion zu gross bemessen ist und aus undurchsichtigem Werkstoff besteht, so dass dieses Hörgerät ein unschönes Gesamtaussehen bietet, welches von der Umwelt in keinem Fall übersehen werden kann.

In der US-A- 2 469 254 wird zwar erstmalig auf ein Hörgerät aus durchsichtigem Kunststoff hingewiesen. Diese Konstruktion ist jedoch wegen der gleichzeitig angestrebten Anpassungsfähigkeit an verschiedene Ohrmuschelgrössen zweiteilig ausgebildet und besitzt zwei verschiebbar ineinander geführte Hälften komplizierter Bauform, die zusammen nur einen Flachtrichter geringer Schallaufnahmefähigkeit ergeben.

Das aus drei Einzelteilen bestehende Hörgerät gemäss US-A- 1 820 107 hat dagegen den Nachteil, dass die Schallauffangtrichter keine plane am Kopf des Trägers anliegende Auflagefläche besitzen, welche die nach vorn gerichtete Position der Schallaufnahmetrichter stabilisieren.

Bei einem weiteren zusammenfaltbaren Hörgerät gemäss US-A- 1 708 257, das nicht auf unterschiedliche Ohrmuschelgrössen einstellbar ist, ist dagegen nicht gewährleistet, dass die Schallauffangtrichter ihre Form im zusammengefalteten und auseinandergespreizten Zustand auch nach längerer Gebrauchszeit beibehalten.

Das einstückig hergestellte Hörgerät gemäss GB-A- 623 791 hat den wesentlichen Nachteil, dass es nur ein Einsteckloch und eine Aufnahmetasche bestimmter Grösse für die Ohrmuschel hat, so dass für abweichende Ohrmuschelgrössen Sondergrössen des Hörgeräts hergestellt werden müssen.

Der Erfindung liegt die Aufgabe zugrunde, unter Vermeidung der aufgeführten Nachteile der in Vergleich gezogenen Hörgeräte, ein Hörgerät gemäss GB-A-6 23 791 dahingehend zu verbessern, dass nur ein Hörgerät einer einzigen Grösse universell bei allen Ohrmuschelgrössen einsetzbar ist, dessen Bauweise bei entscheidend verringerter Sichtbarkeit wesentlich vereinfacht wird.

Gelöst wird diese Aufgabe nach der Erfindung dadurch, dass die Schallauffangtrichter aus elastischem durchsichtigem Kunststoff bestehen und jeder Schallauffangtrichter etwa muschelförmig ausgebildet ist und eine plane, etwa sichelförmige Basisfläche aufweist, deren Ausnehmung dem Verlauf der Ohransatzlinie entspricht und die Ohrmuschel oberseitig hakenartig umgreift.

Nachfolgend wird anhand der Zeichnung eine Ausführungsform der Erfindung näher erläutert.

Es zeigen:

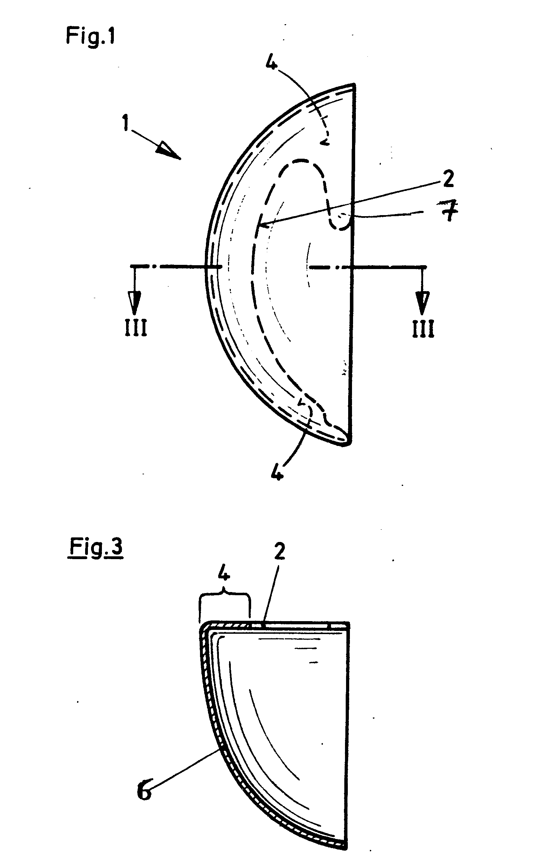

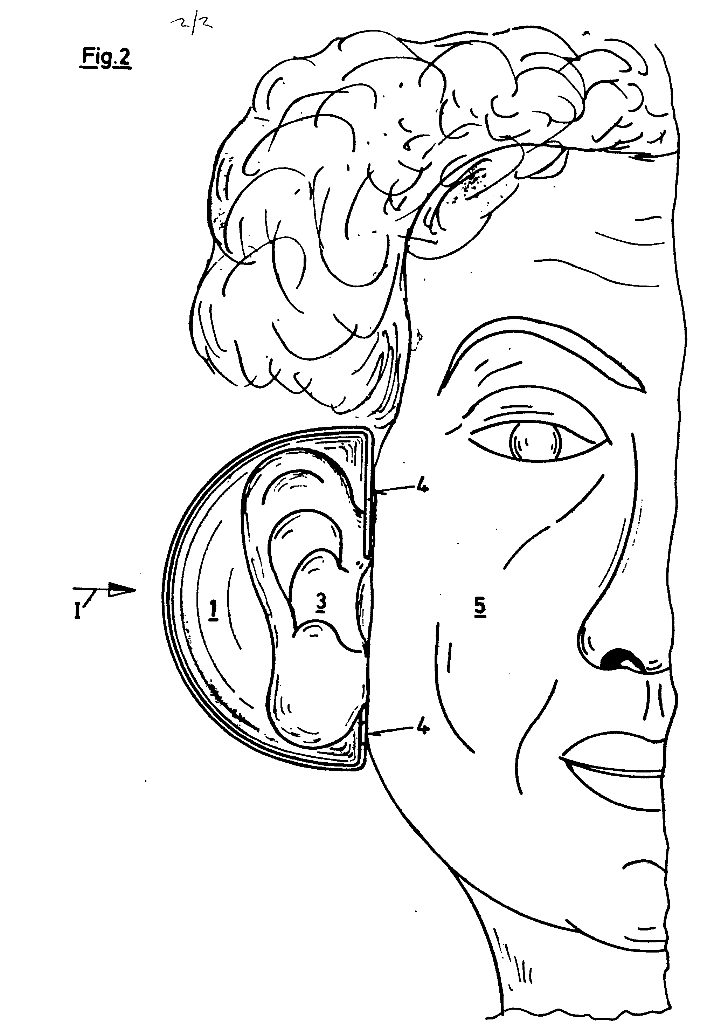

Figur 1: einen Schallauffangtrichter etwa in natürlicher Grösse und in Richtung des Pfeils I gemäss Figur 2 gesehen,

Figur 2: eine andere Ansicht eines auf die natürliche Ohrmuschel aufgesteckten Schallauffangtrichters und

Figur 3: einen Querschnitt durch den Gegenstand nach Figur 1 gemäss Schnittlinie III–III.

Der in der Zeichnung dargestellte etwa muschelförmige Schallauffangtrichter 1 besitzt eine Ausnehmung 2, die derart geformt ist, dass sie der Ohrmuschelansatzlinie entspricht und die Ohrmuschel 3 oberseitig hakenartig (siehe Bezugszeichen 7) umgreift.

Damit ist die Möglichkeit gegeben, eine solche künstliche Ohrmuschel 1 auf die natürliche Ohrmuschel 3 des menschlichen Kopfes 5 aufzustecken, so dass die natürliche Ohrmuschel 3 die künstliche vergrösserte Ohrmuschel 1 trägt.

Der Schallauffangtrichter 1 liegt mit einer sichelförmigen planen Basisfläche 4 auf der Aussenseite des menschlichen Kopfes 5 an und umfasst ausserdem einen über der Basisfläche 4 aufgewölbten Wandungsteil 6.

Akustische Messungen dieses verhältnismässig einfach aufgebauten Hörgerätes haben überraschend gute Resultate ergeben, die eine Verdreifachung der Lautstärke und eine Verdoppelung der Lautigkeit zeigen. Als besonderer weiterer Vorteil ist die Tatsache hervorzuheben, dass die künstlich vergrösserten Ohrmuscheln die durch Hintergrundgespräche verursachten Störgeräusche deutlich abschirmen, was wiederum die nach vorn gerichtete Hörfähigkeit verbessert.

## Patentansprüche

1. Hörgerät mit zwei über die Ohren zu stülpenden, einstückig hergestellten Schallauffangtrichtern aus Kunststoff, dadurch gekennzeichnet, dass

– die Schallauffangtrichter (1,1) aus elastischem durchsichtigem Kunststoff bestehen und

– jeder Schallauffangtrichter (1,1) etwa muschelförmig ausgebildet ist und

– eine plane, etwa sichelförmige Basisfläche (4) aufweist,

– deren Ausnehmung (2) dem Verlauf der Ohransatzlinie entspricht und

– die Ohrmuschel (3) oberseitig hakenartig (7) umgreift.

2. Hörgerät nach Anspruch 1, dadurch gekennzeichnet, dass die Ausnehmung (2) unterhalb der Ohrmuschel (3) eingebuchtet ausgebildet ist.

**Revendications**

1. Appareil auditif ayant deux cornets de réception acoustique monoblocs en matière synthétique à enfoncer sur les oreilles, caractérisé en ce que

– les cornets de réception acoustique (1,1) sont en matière synthétique élastique transparente et

– chaque cornet (1,1) est sensiblement sous la forme d'une coquille, et

– comprend une embase plane (4), sensiblement en forme de croissant,

– dont l'évidement (2) correspond à l'enveloppe du contour de l'oreille et s'accroche par un côté supérieur en forme de crochet (7) au pavillon de l'oreille (3).

2. Appareil auditif selon la revendication 1, caractérisé en ce que l'évidement (2) est échancré en dessous du pavillon de l'oreille (3).

**Claims**

1. A hearing aid having two ear-mounted sound-receiving horns produced in one piece and made of plastics material, characterised in that

– sound-receiving horns (1,1) consist of elastic transparent plastics material and

– each sound-receiving horn (1,1) is approximately shell-shaped in design and

– has a plane approximately crescent-shaped base surface (4),

– the recess (2) of which corresponds to the course of the ear attachment line and

– embraces the ear shell (auricle) (3) at the top in a hook-like manner (7).

2. A hearing aid according to claim 1 characterised in that the recess (2) is designed indented underneath the ear shell (3).

**Fig.1**

**Fig.3**

**Fig.2**

7